# EUROPEAN PATENT SPECIFICATION

(11) **EP 0 330 496 B1**
(45) Date of publication and mention of the grant of the patent: **20.10.1993**
(21) Application number: 89301855.6
(22) Date of filing: 24.02.1989
(51) Int. Cl.: A61K 7/48

(54) **Skin treatment composition**
Hautbehandlungsmittel
Composition pour le traitement de la peau

(30) Priority: 25.02.1988 GB 8804420; 19.08.1988 GB 8819737
(43) Date of publication of application: 30.08.1989
(73) Proprietor: BEECHAM GROUP PLC, Brentford, Middlesex TW8 9BD (GB)
(72) Inventor: Batt, Margaret Dorothy, Weybridge Surrey KT13 ODE (GB)
(74) Representative: West, Vivien

(56) References cited:
- EP-A- 0 094 771
- EP-A- 0 253 393
- EP-A- 0 255 364
- WO-A-86/06275
- GB-A- 1 239 965
- CHEMICAL ABSTRACTS, vol. 98, 1983, page 358, no. 132150g, Columbus, Ohio, US; & ES-A-506 622 (TRAUMHAFT, S.A.) 01-10-1982
- CHEMICAL ABSTRACTS, vol. 73, 1970, page 216, no. 59219p, Columbus, Ohio, US; H. VALFRE: "Stability of vitamin A in cosmetic aerosol formulations" & REV. QUIM. IND. (RIO DE JANEIRO) 1970, 39(453), 14 (PORT)
- PATENT ABSTRACTS OF JAPAN, vol. 13, no. 227 (C-600)[3575], 25th May 1989; & JP-A-1 40412 (POLA CHEM. IND. INC.) 10-02-1989

## Description

This invention relates to the use of topical skin treatment compositions containing esters of Vitamin A (retinol) that are suitable for application to photoaged skin, that is skin damaged by UV exposure.

It is known that retinoic acid is suitable for treating a series of skin ailments, for example acne, and also for long term use to reduce wrinkling. It is also found that topical applications have significant effects on the texture and characteristics of photoaged skin.

US Patent No. 4,603,146 describes and claims a method of retarding and reversing the loss of collagen fibres, abnormal changes in elastic fibres, deterioriation of small blood vessels and the formation of abnormal epithelial growths in the skin by treating sundamaged human skin with retinoic acid in an emollient vehicle. However, in the doses used for such treatment, retinoic acid can cause severe irritation problems.

It has been found that many of the epidermal effects of retinoic acid described above are present in the esters of retinol, without the irritation response shown by the acid.

In particular, retinyl propionate has been shown to stimulate epidermal mitotic activity, thicken the viable cell layers of the epidermis and both induce a granular layer and normalize keratin from the para to the orthokeratotic form.

In particular, retinyl propionate has been shown to stimulate epidermal mitotic activity, and both induce a granular layer and normalize keratin from the para to the orthokeratotic form.

Further studies show that topical application of retinyl propionate is active in stimulating the repair of UV damaged dermal connective tissue.

Accordingly the invention provides use of a C₂₋₁₆ alkanoyl ester of retinol for the manufacture of a topical composition for use in the treatment of photoaged human skin.

In particular, there is provided the use of a skin treatment composition, comprising a topically acceptable oil-in-water or water-in-oil emulsion base and an effective amount of a C₂₋₁₆ alkanoyl ester of retinol, in the treatment of photoaged human skin.

A further aspect of the invention provides a method of treatment of photoaged human skin, which method comprises the administration thereto of an effective amount of a C₂₋₁₆ alkanoyl ester of retinol.

Preferably, the composition according to the invention comprises a C₂₋₁₀ alkanoyl ester of retinol.

Preferably, said composition comprises a C₃₋₇ straight chain, branched chain, or cyclo alkanoyl ester of retinol, for example retinyl propionate, butyrate, cyclopentanecarboxylate, pivalate, valerate, hexanoate or heptanoate.

The composition is suitable for use in the stimulation of epidermal mitosis and/or the treatment of photoaged human skin.

The compositions according to the present invention are characterized by improved activity compared to the retinyl esters, particularly the palmitate, when formulated in a conventional emulsion base such as those described in GB-A-1,489,133.

A further aspect of the invention provides a method of treatment of photoaged human skin, which method comprises the administration thereto of an effective amount of at least one ester of retinol or of a composition of the invention.

The invention also provides use of an ester of retinol for the manufacture of a topical composition for use in the treatment of photoaged human skin.

The ester of retinol is typically an ester of a fatty acid, for example an essential fatty acid such as linoleic acid.

Preferably, the composition according to the invention comprises a C₂₋₁₆ alkanoyl ester, more preferably a C₂₋₁₀ alkanoyl ester, of retinol.

Preferably, said composition comprises a C₃₋₇ straight chain, branched chain, or cyclo alkanoyl ester of retinol, for example retinyl propionate, butyrate, cyclopentanecarboxylate, pivalate, valerate, hexanoate or heptanoate.

Preferably, the composition comprises from 500 to 15,000, more particularly from 1,000 to 10,000 I Ug⁻¹, or more suitably from 2,000 to 5,000 I Ug⁻¹ of the retinyl ester or mixture of esters.

The preferred emulsion base is an oil-in-water emulsion base containing from 20 to 40% by weight of oil.

An emulsifier is preferably used in the composition of the invention, and this may be an anionic, cationic or non-ionic type. However, cationic emulsifiers can cause skin irritation when used, and non-ionic emulsifiers are, therefore, preferred.

It has been found that the proportion of non-ionic emulsifier in the compositions of the invention is not critical, but at least 1% by weight is preferred to give a stable emulsion. Suitably an amount in the range 2% to 12%, more suitable 4% to 10% is preferred.

Examples of non-ionic emulsifiers suitable for inclusion in the present compositions include: sorbitan monostearate, glyceryl monostearate, polysorbates, polyethylene derivatives of fatty alcohols, sucrose esters, lecithins, glyceride esters, methylglucoside esters and dimethicone copolyols.

Examples of oils suitable for inclusion in the present compositions include: volatile linear iso-paraffins, acyclic dimethylpoly siloxanes, cyclic dimethylpoly siloxanes, mineral oils, vegetable oils, synthetic fatty acid esters, fatty alcohols, lanolin and its derivatives and carbofluorated oils.

Alternatively, a water-in-oil emulsion base may be formulated containing 35-65% by weight of oil.

The preferred emulsifier in a w/o emulsion is a non-ionic co-polymer suitably in an amount in the range of 1-3%, but at least 1% must be present for a stable emulsion to be formed.

Examples of oils and waxes suitable for inclusion in a w/o composition include, mineral oil, ceresin wax, beeswax, volatile linear isoparaffin, cyclomethicones and hydroxyoctacosanyl hydroxystearate.

The compositions according to this invention may also include anti-inflammatory materials such as Allantoin(2,5-dioxo-4-imidazolidinyl-urea), Bisabolol (2S, 4'S) 6-methyl-2-(4'-methylcyclohex-3- enyl)hept-5-en-2-ol, and beta glycerrhetinic acid.

Alternatively, as indicated above, an anhydrous non-oily composition may be formulated, wherein water is excluded from the preparation, using a volatile oil such as silicone DC 345 with which an anhydrous alcohol is optionally admixed.

Humectant materials may also be included in the compositions of this invention which will enhance the moisturizing effect by attracting moisture to the skin. Examples of suitable humectants include Lubrajel (polyglyceryl methacrylate plus propylene glycol), sodium hyaluronate, propylene glycol, glycerol, sorbitol and sodium pyrrolidone carboxylate.

The compositions according to this invention should desirably include an anti-oxidant effective in preventing oxidation of the retinol ester and consequent reduction in the activity of the composition. Some anti-oxidants are effective in this respect but themselves oxidise to give a noticeable yellowing of the cream.

Anti-oxidants which are particularly suitable for incorporation include butylated hydroxytoluene (BHT), (2,3-di-tert-butyl-p-cresol), butylated hydroxyanisole (BHA), (2-tert-butyl-4-hydroxyanisole or 3-tert-butyl-4-hydroxyanisole), or a mixture of these. Alternatively DL alphatocopherol (Vitamin E) esters could be used.

Accordingly in another of its aspects, the invention includes a composition in accordance with the invention including BHT and/or BHA as anti-oxidant.

The retinol ester of the composition may be dispersed in the emulsion in the form of a 'solubilised' mixture. Such mixtures are generally solutions of the retinol ester in hydrophilic organic solvents such as glycerine and/or propylene glycol, together with a surfactant such as Tween 80.

The skin-care compositions according to the invention, which comprise a topically acceptable oil-in-water or water-in-oil emulsion base and from 500 to 15,000 IUg⁻¹ of retinyl propionate may be prepared by mixing the emulsion base with the retinol ester.

Preferably the retinol component is added to the emulsion base with stirring at a temperature of less than 50°C, suitably 35°C.

The following Examples are prepared in accordance with the present invention:

### Example 1

The following o/w emulsion was prepared in the conventional manner:

| | % w/w |
|---|---|
| Glyceryl Stearate and Peg 100 Stearate | 6.00 |
| Ceteary Octanoate | 1.50 |
| Octylpalmitate | 5.00 |
| Propylene Glycol | 3.00 |
| Glycerin | 5.00 |
| Cyclomethicone | 5.00 |
| Retinyl Propionate | 0.15 |
| Parabens | 0.40 |
| Imidazolidinyl Urea | 0.30 |
| Fragrance | 0.50 |
| BHT | 0.20 |
| Water | to 100 |

### Example 2

| | % w/w |
|---|---|
| Glyceryl Stearate | 4.00 |
| Sucrose Distearate | 0.50 |
| Carbofluorated Oils | 3.00 |
| Isocetyl Stearate | 5.00 |
| Propylene Glycol Dioctoate | 4.00 |
| Acetate DL Alpha Tocopherol | 0.50 |
| Water Soluble Retinol Ester | 2000-7000 IUg⁻¹ |
| Propylene Glycol | 3.00 |
| Preservatives | 0.50 |
| Fragrance | 0.50 |
| Deionised Water | to 100 |

### Example 3

| | % w/w |
|---|---|
| Hydrogenated Tallow Glyceride Citrate | 5.00 |
| Glyceryl Palmitate Lactate | 0.50 |
| Lecithin | 1.00 |
| Mineral Oil | 5.00 |
| Hydrogenated Polyisobutene | 5.00 |
| Cyclomethicone | 5.50 |
| Water Soluble Retinol Ester | 500-5000 IUg⁻¹ |
| DL Alpha Tocopherol Acetate | 0.50 |
| Glycerine | 5.00 |
| Preservatives | 0.63 |
| Fragrance | 0.50 |
| Deionised Water | to 100 |

### Example 4

| | % w/w |
|---|---|
| Sucrose Distearate | 4.50 |
| Sucrose Stearate | 0.50 |
| Octyl Palmitate | 5.80 |
| Propylene Glycol Dicaprylate/Dicaprate | 5.00 |
| BHT | 0.20 |
| Sorbitol | 4.00 |
| Water Soluble Retinol Ester | 500-7000 IUg⁻¹ |
| Preservatives | 0.70 |
| Fragrance | 0.50 |
| Deionised Water | to 100 |

### Example 5

| | % w/w |
|---|---|
| Dimethicone Copolyol | 1.00 |
| Cyclomethicone | 19.00 |
| Stearoxy Dimethicone | 0.20 |
| Hydroxyethylcellulose | 0.05 |
| Glycerine | 3.00 |
| Water Soluble Retinol Ester | 500-7000 IUg⁻¹ |
| BHT | 0.20 |
| Preservatives | 0.40 |
| Fragrance | 0.50 |
| Deionised Water | to 100 |

### Example 6

The following w/o emulsion was prepared in the conventional manner:

| | % w/w |
|---|---|
| Mineral Oil | 10.00 |
| Cyclomethicone | 10.00 |
| Ceresin Wax | 2.00 |
| PEG22/Dodecyl Polymer | 1.00 |
| Hydroxyoctacosanyl Hydroxystearate | 1.00 |
| Sorbitol (70% aqueous sol.) | 9.00 |
| Butyl Paraben | 0.06 |
| 2-Bromo-2 Nitropropan-1,3-Diol | 0.02 |
| Esters of Ethyl Propyl and Butyl Esters of P-Hydroxybenzoic Acid | 0.30 |
| Butylated Hydroxytoluene | 0.20 |
| Benzophenone-3 | 2.00 |
| Vitamin A Propionate | 0.14 |
| Perfume | 0.20 |

### Example 7

| Silicone Emulsion | |
|---|---|
| | % w/w |
| *Silicone Emulsion Q2.322 | 10.00 - 20.00 |
| *Silicone DC 345 | 10.00 |
| Sodium Chloride | 1.00 |
| Retinyl Propionate | 0.15 |
| Perfume | qs |
| Preservative | qs |
| Deionised Water | to 100.00 |

| | |
|---|---|
| *Q2.3225C - volatile silicone self emulsifying CTFA name Cyclomethicone (and) Dimethicone Copolyol | |
| *DC 345 CTFA name Cyclomethicone | |

### Example 8

| Anhydrous non-oily skin tonic | |
|---|---|
| | % w/w |
| Silicone DC 345 | 34.00 - 98.85 |
| Propylene Glycol | 1.00 |
| Retinyl propionate | 0.15 |
| Perfume | qs. |
| Dyes | qs. |
| Anhydrous alcohol (optional) | to 100.00% |

The active retinyl propionate is slowly dissolved in the silicone oil and the remaining ingredients are incorporated in a conventional manner.

### Example 9

Retinyl palmitate was substituted for retinyl propionate in the skin tonic of Example 8.

## Claims

1. Use of an ester of retinol for the manufacture of a composition for the treatment of photodamaged skin.

2. Use of an ester of retinol for the manufacture of a composition for stimulating the repair of U.V damaged dermal connective tissue.

3. The use as claimed in claim 1 or 2 wherein the composition comprises a topically acceptable anhydrous base and an ester of effective amount of at least one ester of retinol.

4. The use as claimed in any of claims 1 to 3 wherein the ester is a C₂₋₁₆ alkanoyl ester of retinol.

5. The use as claimed in claim 4 wherein the C₂₋₁₆ alkanoyl ester is retinyl palmitate or retinol propionate.

6. The use as claimed in Claim 1 to 5 wherein the ester is present in a topically acceptable anhydrous emulsion and comprises from 500 to 15,000 I.u g⁻¹ of the composition.

## Patentansprüche

1. Verwendung eines Retinolesters zur Herstellung einer Zusammensetzung zur Behandlung lichtgeschädigter Haut.

2. Verwendung eines Retinolesters zur Herstellung einer Zusammensetzung zur Anregung der Wiederherstellung von U.V.-Lichtgeschädigtem Unterhaut-Bindegewebe.

3. Verwendung nach Anspruch 1 oder 2, wobei die Zusammensetzung eine lokal verträgliche, wasserfreie Base und eine wirksame Menge mindestens eines Retinolester umfaßt.

4. Verwendung nach einem der Ansprüche 1 bis 3, wobei der Ester ein C₂₋₁₆-Alkanoylester des Retinols ist.

5. Verwendung nach Anspruch 4, wobei der C₂₋₁₆-Alkanoylester Retinylpalmitat oder Retinylpropionat ist.

6. Verwendung nach Anspruch 1 oder 5, wobei der Ester in einer lokal zulässigen, wasserfreien Emulsion vorliegt und 500 bis 15 000 I.U.g⁻¹ der Zusammensetzung umfaßt.

## Revendications

1. Utilisation d'un ester de rétinol pour la fabrication d'une composition pour le traitement d'une peau photo-endommagée.

2. Utilisation d'un ester de rétinol pour la fabrication d'une composition pour stimuler la réparation d'un tissu conjonctif dermique endommagé par des UV.

3. Utilisation suivant la revendication 1 ou 2, dans laquelle la composition comprend une base anhydre acceptable du point de vue topique et une quantité efficace d'au moins un ester de rétinol.

4. Utilisation suivant l'une quelconque revendications 1 à 3, dans laquelle l'ester est un ester alcanoylique en C₂₋₁₆ du rétinol.

5. Utilisation suivant la revendication 4, dans laquelle l'ester alcanoylique en C₂₋₁₆ du rétinol est le palmitate de rétinyle ou le propionate de rétinyle.

6. Utilisation suivant l'une quelconque revendications 1 à 5, dans laquelle l'ester est présent dans une émulsion anhydre acceptable du point de topique et représente de 500 à 15.000 U.I/g de la composition.
